# EUROPEAN PATENT APPLICATION

(11) **EP 0 897 978 A2**
(43) Date of publication of application: **24.02.1999**
(21) Application number: 98115595.5
(22) Date of filing: 19.08.1998
(51) Int. Cl.: C12N 15/10, C07H 1/08, C12P 19/34, C12Q 1/68

(54) **Zirconium oxide and related compounds for purification of nucleic acids**

(30) Priority: 22.08.1997 US 916531
(71) Applicant: Becton, Dickinson and Company, Franklin Lakes, New Jersey 07417-1880 (US)
(72) Inventor: Woodard, Daniel L., Raleigh, North Carolina 27613 (US); Beyer, Wayne F., Jr., Bahama, North Carolina 27503 (US)
(74) Representative: Rambelli, Paolo

(57) **Abstract**

The present invention relates to unique compositions which bind nucleic acids. The compositions are useful in processes for purifying nucleic acid from a sample.

## Description

### BACKGROUND OF THE INVENTION

The present invention relates generally to purification of nucleic acids by solid phase extraction, and more specifically to zirconium oxide and related surfaces which are capable of binding and eluting nucleic acids under suitable conditions.

The preparation and purification of high-purity double-stranded (ds) plasmid DNA, single-stranded (ss) phage DNA, chromosomal DNA, agarose gel-purified DNA fragments and RNA is of critical importance in molecular biology. Ideally, a method for purifying nucleic acids should be simple, rapid and require little, if any, additional sample manipulation. Nucleic acids rendered by such a method should be immediately amenable to transformation, restriction analysis, litigation or sequencing. A method with all of these features would be extremely attractive in the automation of nucleic acid sample preparation, a goal of research and diagnostic laboratories.

Typically, the preparation of plasmid DNA from crude alcohol precipitates is laborious, most often utilizing CsCl gradients, gel filtration, ion exchange chromatography, or RNase, proteinase K and repeated alcohol precipitation steps. These methods also require considerable downstream sample preparation to remove CsCl and other salts, ethidium bromide and alcohol. Similar arguments extend when using any of these methods for purifying DNA fragments. A further problem with these methods is that small, negatively-charged cellular components can copurify with the DNA. Thus, the DNA can have an undesirable level of contamination.

Nucleic acids can also be purified using solid phases. Conventional solid phase extraction techniques have utilized surfaces which either (1) fail to attract and hold sufficient quantities of nucleic acid molecules because of surface design to permit easy recovery of the nucleic acid molecules during elution, or (2) excessively adhere nucleic acid molecules to the surface, thereby hindering recovery of the nucleic acid molecules during elution. Conventional metal surfaces which cause these problems when utilized in solid phase extraction include silica surfaces such as glass and Celite. Adequate binding of nucleic acids to these types of surfaces can be achieved only by utilizing high concentrations of chaotropes or alcohols which are generally toxic, caustic, and/or expensive. For example, it is known that DNA will bind to crushed glass powders and to glass fiber filters in the presence of chaotropes. The chaotropic ions typically are washed away with alcohol, and the DNAs are eluted with low-salt solutions or water. Importantly, RNA and protein do not bind. However, a serious drawback in the use of crushed glass powder is that its binding capacity is low. In addition, glass powders often suffer from inconsistent recovery, incompatibility with borate buffers and a tendency to nick large DNAs. Similarly, glass fiber filters provide a nonporous surface with low DNA binding capacity. Other silicas, such as silica gel and glass beads, are not suitable for DNA binding and recovery. Currently, the solid phase of choice for solid phase extraction of DNA is Celite such as found in Prep-A-Gene™ by Bio-Rad Laboratories. As with the crushed glass powders, high concentrations of chaotropes are required for adequate binding of the DNA to the Celite.

There are numerous protocols for purifying DNA. For example, U.S. Patent 4,923,978 discloses a process for purifying DNA in which a solution of protein and DNA is passed over a hydroxylated support and the protein is bound and the DNA is eluted. U.S. Patent 4,935,342 discloses purification of DNA by selective binding of DNA to anion exchangers and subsequent elution. U.S. Patent 4,946,952 discloses DNA isolation by precipitation with water-soluble ketones. A DNA purification procedure using chaotropes and dialyzed DNA is disclosed in U.S. Patent 4,900,677.

Diatoms have also been utilized for purification of nucleic acids as evidenced by U.S. Patent No. 5,234,809 to Boom et al. and U.S. Patent No. 5,075,430 to Little et al.

### SUMMARY OF THE INVENTION

In order to provide a more effective and efficient technique for the purification of nucleic acids, the present invention relates to a zirconium oxide composition which may be modified by refluxing zirconium oxide with sodium hydroxide. This zirconium oxide composition is particularly useful for purification of nucleic acids from other cellular components. Other related compositions which were also found to be useful in such nucleic acid purification methods include hafnium oxide and aluminum oxide. All of these metal oxide compositions are useful for nucleic acid purification in either hydrated or unhydrated form.

Such a nucleic acid purification process involves exposure of the zirconium oxide or related composition to a sample containing cellular components and then separating the composition from the sample. Nucleic acids in the sample are bound to the zirconium oxide or related composition, and can be recovered by elution with a suitable elution buffer.

When used in nucleic acid purification processes, the zirconium oxide or related composition of the present invention may be in the form of particles or beads for ease of packaging in a kit format.

### DETAILED DESCRIPTION OF THE INVENTION

The present invention relates to unique compositions of matter. The compositions of matter include a zirconium oxide composition, a hafnium oxide composition and an aluminum oxide composition, all of which bind nucleic acids. The nucleic acid bound to these compositions may be eluted therefrom.

The compositions of the present invention may be modified by refluxing a starting material, such as zirconium oxide, hafnium oxide or aluminum oxide with a suitable alkaline substance such as sodium hydroxide. Suitable starting materials for use in the refluxing reaction are commercially available from numerous sources in a particulate or pelleted form. However, any form or shape of starting material is suitable for refluxation to produce a hydrated form of the composition of the present invention.

The refluxing of the starting material is with a suitable alkaline substance. Such a substance is a composition that contributes to the resultant hydrated composition of the present invention being sufficiently electropositive to bind nucleic acids which are relatively electronegative. Most strong bases in water will be suitable alkaline substances for refluxing with starting material to yield the hydrated composition of the present invention. Examples of such suitable alkaline substances include sodium hydroxide, potassium hydroxide or any other base which generates a hydroxide ion.

Subsequent reactions to which the bound nucleic acid may be subjected may determine which alkaline substance is preferred. For example, because sodium ions are believed to inhibit Strand Displacement Amplification ("SDA") reactions, a hydroxide ion donor other than sodium hydroxide, such as potassium hydroxide, may be preferred if the nucleic acids bound to the hydrated zirconium silicate composition are to be amplified by SDA.

The amount of alkaline substance used in the refluxing reaction is generally about 0.1M equivalents to about 5M equivalents of the amount of starting material used in the reaction.

The refluxing reaction used to produce the hydrated compositions of the present invention is generally conducted for about 18 to about 96 hours at about 100°C (*i.e.* reflux). Following such refluxation, the resultant hydrated composition may optionally be filtered and washed with aqueous acid such as sulfuric acid or hydrochloric acid. An alternative option is to use such aqueous acids to acidity the refluxed suspension of hydrated composition to a pH of 6.0 or lower, and then filter and wash the hydrated composition with a suitable wash buffer such as water and/or acetone.

The hydrated composition produced by the above-described reflux reaction binds nucleic acid. It is believed that the binding of nucleic acid is due at least in part to the attraction of the electropositive hydrated composition for electronegative nucleic acids. More specifically, the positively charged atoms, for instance Hf⁴⁺ from HfO₂, Zr⁴⁺ from ZrO₂ and Al³⁺ from Al₂O₃ provide sufficient electropositivity to the hydrated composition to interact with the negatively charged-phosphates of the nucleic acids, thus causing binding.

As set forth in greater detail in the Examples below, the ability of the hydrated compositions as well as the unhydrated compositions of the present invention to bind nucleic acids is quantifiable by the percentage of nucleic acid bound to the surface from an otherwise clean simple (*i.e.* without the non-target nucleic acids) under certain conditions. Due to the generally small volumes of samples from which nucleic acids are purified, the percentage of bound nucleic acid is based on a small amount of hydrated or unhydrated composition (*i.e.* 2.5mg), and a total amount of nucleic acid which is generally found in a typical sample from which nucleic acid is purified (*i.e.* about 10⁴ target nucleic acid molecules).

Thus, in order to have some utility in a process of purifying nucleic acid from a sample, a nucleic acid binding composition present in an amount of about 2.5mg in a sample containing about 10⁴ target nucleic acid copies should bind at least about 85 percent of the target nucleic acid copies. As shown in the Examples below, the hydrated and unhydrated composition of the present invention meet this requirement.

The nucleic acids which are bound by the compositions of the present invention include DNA and RNA obtained from any source, including but not limited to crude cell extracts, biological fluids, phage supernatants, agarose gels and radiolabelling reactions. The nucleic acid, particularly DNA can be double-stranded, single-stranded, circular or linear, and can be variable in size. Conventional techniques for obtaining nucleic acid from any source, well known in the art, are utilized to prepare the nucleic acid for purification. Typical procedures for obtaining nucleic acid end with a suspension of the nucleic acid in solution. For isolation of nucleic acid from biological samples, see, e.g., Harding, J.D. et al., Nucleic Acids Research 17:6947 (1989) and Marko, M.A. et al., Analytical Biochemistry 121:382 (1982). Procedures for isolation of plasmid DNA can be found in Lutze, L.H. et al., Nucleic Acids Research 20:6150 (1990). Techniques for extraction of double-stranded DNA from biological samples can be found in Yamada, O. et al., Journal of Virological Methods 27:203 (1990). Most DNA solutions comprise the DNA in a suitable buffer such as TE (Tris-EDTA), TEA (409 mm Tris-acetate, 1 mm EDTA) buffer, or a lysate. See also Sambrook, J. et al., Molecular Cloning: A Laboratory Manual, 2nd Ed., Cold Spring Harbor Laboratory Press, New York (1989).

Once the nucleic acid is obtained in a suitable sample form, generally suspension or solution, a composition of the present invention is exposed to the sample for a period of time sufficient for nucleic acid in the sample to bind to the composition. Generally, such exposure is for a time period of about 30 seconds to about 60 minutes at a temperature of about 0°C to about 25°C. A suitable binding buffer, such as, for example, water and acidic buffers with pHs below about 7.0, may be used to assist the binding of nucleic acid to the composition.

The composition with nucleic acid bound thereto is then separated from the sample. Separation is generally accomplished by centrifugation or filtration.

The composition may then be washed, and the nucleic acid may be recovered from the composition. Washing is generally performed with low molecular weight alcohols or other suitable washing solutions such as 80%/20% ethanol/50mM Tris, pH 7.0, water or any low pH buffer.

The recovery of bound nucleic acid from composition is generally conducted by elution with a suitable elution buffer. Suitable elution buffers include most basic buffers with pHs above about 7.0 such as, for example, any low salt buffer such as 50mM Tris, KPI and KPDG. Preferably, the elution buffer will have minimal interference with processes to which the recovered nucleic acid may be subjected and have minimal negative environmental effects. Typically, elution recovery of nucleic acids from composition is conducted over a time period of about one minute to about 60 minutes at a temperature from about 4°C to about 95°C. One way in which such elution may be conducted is to flow the elution buffer past the composition with nucleic acid bound thereto to cause the elution of nucleic acid. Alternatively, the composition with nucleic acid bound thereto may be placed into the elution buffer, and then separated from the elution buffer in order that the nucleic acid may be recovered from the elution buffer.

The nucleic acid obtained by purification with a composition of the present invention may be used without further manipulation for restriction enzyme digestion, cloning, sequencing, diagnostics and the like. The high quality of nucleic acid prepared with the present invention and the speed with which nucleic acid is purified with minimal downstream processing mean that the composition can be useful in the automation of nucleic acid sample preparation.

One particularly useful form for the compositions of the present invention for automated nucleic acid sample preparation is as particles. Particulation of the composition provides maximal surface area for binding of nucleic acid. The particles may be of various shapes, including for example, spheres, cubes, oval, capsule-shaped, tablet-shaped, non-descript random shaped, etc., and may be of uniform shape or non-uniform shapes. Whatever the shape of a particle, its diameter at its widest point is generally in the range of from about 0.1 mm to about 0.15 mm. Pellets or beads of a generally spherical shape are the preferred particulate form of the composition of the present invention.

The hydrated forms of the compositions of the present invention are also particularly useful in a particle form because of their density range of about 1.0mg/uL to about 4.0mg/uL with a preferred value of about 3.3mg/uL. For comparison, hydrated Celite has a density of about 0.4mg/uL. This unusually high density of the hydrated forms of the compositions of the present invention renders it easier to separate the particles of such compositions with bound nucleic acid from the sample by centrifugation. Also, due to the density of the particles, the pellet formed by the centrifugation is tighter than with less dense compounds allowing a longer time, if desired, between centrifugation and supernatant (sample) removal, as well as an ability to remove more of the supernatant (sample) without dislodging portions of the pellet. The ability to remove more of the supernatant is particularly important as more of the cellular debris and binding buffer are removed, both of which may inhibit or interfere with subsequent processing of the purified nucleic acids.

Particulate forms of the compositions of the present invention are also advantageous due to their ease of use in kits. Particles can be easily and efficiently packaged in any suitable container as a kit or part of a kit for purification of nucleic acids from samples. Suitable containers for packaging particles of the compositions of the present invention include vials, tubes, evacuated tubes or containers, and other containers to which a sample containing nucleic acids may be added. Such kits may also include other containers of binding buffer, wash buffer and/or elution buffer and any other components desired as part of a process to purify nucleic acids from a sample.

The following examples illustrate specific embodiments of the invention described in this document. As would be apparent to skilled artisans, various changes and modifications are possible and are contemplated within the scope of the invention described.

### EXAMPLE 1

### Production of Hydrated Zirconium Oxide Composition

A four gram aliquot of zirconium oxide beads from Aldrich was added to 80mL of water containing 5.2 grams sodium hydroxide (NaOH). The resulting suspension was refluxed for 68 hours, and then cooled to room temperature and centrifuged at 1800 rpm for 3.5 minutes, and the supernatant removed and discarded. The suspension was then rendered acidic (pH of about 2.0) by addition of 20ml of 1% sulfuric acid (H₂SO₄) and 5mL of 10% H₂SO₄. Alternatively, the solid is isolated from solution, and then added to the acidic solution. This acidic suspension was rocked at room temperature for one hour, and then centrifuged at 1800 rpm for 3.5 minutes, and the supernatant removed and discarded. To each tube, 20ml of water was added, and the suspensions vortexed and filtered followed by washes with 100ml of water and 100ml of acetone. Finally, the powders were air dried for one hour and then oven dried for 16 hours at 100°C.

The resultant hydrated zirconium oxide powders were sufficiently electropositive to bind nucleic acids which could then be easily eluted therefrom as shown in the subsequent Examples.

### EXAMPLE 2

### Production of Hydrated Hafnium Oxide Composition

A four gram aliquot of hafnium oxide beads from Aldrich was added to 80ml of water containing 3.04 grams sodium hydroxide (NaOH). Then, the same procedure set forth in Example 1 was followed to yield hydrated hafnium oxide beads sufficiently electropositive to bind nucleic acids which could then be easily eluted therefrom as shown in subsequent Examples.

### EXAMPLE 3

### Production of Hydrated Aluminum Oxide Composition

A four gram aliquot of aluminum oxide beads from Aldrich was added to 80ml of water containing 6.28 grams sodium hydroxide (NaOH). Then, the same procedure set forth in Example 1 was followed to yield hydrated aluminum oxide beads sufficiently electropositive to bind nucleic acids which could then be easily eluted therefrom as shown in subsequent Examples.

### EXAMPLE 4

### Binding and Elution of Nucleic Acid To and From Hydrated Compositions

This experiment was performed to demonstrate the nucleic acid binding and elution characteristics of hydrated and unhydrated zirconium oxide, hafnium oxide and aluminum oxide.

The compositions of Example 1, 2 and 3 (hydrated zirconium oxide, hydrated hafnium oxide and hydrated aluminum oxide, respectively) as well as unhydrated forms of these three compositions were mixed with 200µL water followed by the addition of 1µL of 10⁶ P³² labeled *M. tuberculosis* DNA. The resulting suspensions were incubated at room temperature 30 minutes on a rotator device. Following centrifugation, the supernatents were removed and added to 10mL of scintillation fluid for counting. 200µL of water was added to the remaining pellets and the resulting mixtures were incubated at 65°C for 30 minutes. Following centrifugation the supernatents were counted and the elution step repeated.

The results are shown below.

### Conclusions

Although the unhydrated zirconium oxide, hafnium oxide and aluminum oxide compositions are useful for nucleic acid purification, their hydrated counterparts are better for such purpose because of greater binding and elution of nucleic acid under comparable conditions. Also, the hydrated counterparts showed comparable nucleic acid purification properties to hydrated zirconium silicate compositions.

### EXAMPLE 5

### Increasing pH Effect or Elution of Nucleic Acid from Hydrated Compositions

This experiment was performed to determine if nucleic bound to hydrated zirconium silicate beads (comparative composition of Example 4) would elute at various pHs.

The same protocol as used in Example 4 was used to bind *M. tuberculosis* DNA to 10mg of beads of hydrated zirconium silicate, except that following the first centrifugation, in addition to water, different concentrations of NaOH were used as elution buffers. The results are set forth below.

| **REACTION No.** | **ELUTION BUFFER** | **CPM SUPER BINDING** | **CPM SUPER ELUTION #1** | **CPM SUPER ELUTION #2** | **% DNA BOUND** | **% DNA ELUTED** |
|---|---|---|---|---|---|---|
| 1 | Water | 461 | 542 | 234 | 100 | 1 |
| 2 | NaOH pH10 | 422 | 55140 | 9767 | 100 | 93 |
| 3 | NaOH pH12 | 369 | 70322 | 3096 | 100 | 100 |
| 4 | NaOH 1N | 303 | 71518 | 3755 | 100 | 100 |

| **CONTROL NO SURFACE ADDED** | | | | | | |
|---|---|---|---|---|---|---|
| 5 | | 69619 | | | | |

### Conclusions

There does seem to be a significant correlation between pH of the elution buffer and percentage DNA eluted, although other factors may be involved. Also, due to the similar nucleic acid purification properties of the other hydrated compositions as shown in Example 4, it is expected that such hydrated compositions would show similar correlation between pH of the elution buffer and percentage DNA eluted.

### EXAMPLE 6

### Effects of Elution Buffers on Nucleic Acid Bound to Hydrated Compositions

This experiment was performed to determine if nucleic acid bound to hydrated zirconium silicate beads would elute in various buffers.

200 uL water was added to a centrifuge tube containing 10mg of hydrated zirconium silicate beads followed by 1uL of DNA (10⁴ copies of *M. tuberculosis* genomic DNA). The resulting suspension was incubated at room temperature on a rotator device for 30 minutes. Following centrifugation, the supernate was removed and added to 10mL scintillation fluid and counted. 200uL of buffer was added to the pellet. The resulting suspension was incubated at 65°C for 30 minutes. Following centriffugation the supernate was removed and counted as above and the elution step repeated. The results are shown below.

| **REACTION NO.** | **ELUTION BUFFER** | **CPM SUPER BINDING** | **CPM SUPER ELUTION #1** | **CPM SUPER ELUTION #2** | **% DNA BOUND** | **% DNA ELUTED** |
|---|---|---|---|---|---|---|
| 1 | Water | 565 | 668 | 266 | 100 | 1 |
| 2 | NaOH pH7.2 | 573 | 4053 | 5254 | 100 | 11 |
| 3 | NaHCO3 pH9.4 | 579 | 61630 | 3996 | 100 | 81 |
| 4 | 25MMKPI pH7.6 | 414 | 63961 | 5055 | 100 | 85 |
| 5 | KPDG pH7.6 | 475 | 64701 | 2564 | 100 | 83 |

| **CONTROL NO SURFACE ADDED** | | | | | | |
|---|---|---|---|---|---|---|
| 6 | | 81466 | | | | |

### Conclusions

The buffers used elute much more DNA than does water. As in Example 5, due to the similar nucleic acid purification properties of the other hydrated compositions as shown in Example 4, it is expected that such hydrated compositions would show similar elution characteristics to the hydrated zirconium silicate.

### EXAMPLE 7

### Nucleic Acid Binding Conditions for Hydrated Compositions

This experiment was performed to determine preferred binding conditions for nucleic acid to 5mg of hydrated zirconium silicate composition.

The same protocol as used in Example 4 was followed except that binding buffers and incubation times were used as shown below, and elution centrifugations were not performed. The results are shown below.

| **REACTION NO.** | **BINDING BUFFER** | **BINDING TEMP C** | **CPM SUPER BINDING** | **% DNA BOUND** |
|---|---|---|---|---|
| 1 | H20 | 4 | 630 | 97 |
| 2 | H20 | 25 | 506 | 98 |
| 3 | H20 | 37 | 1098 | 96 |
| 4 | 25MM KPI pH 7.6 | 4 | 21352 | 0 |
| 5 | 25MM KPI pH 7.6 | 25 | 22045 | 0 |
| 6 | 25MM KPI pH 7.6 | 37 | 20302 | 0 |
| 7 | HCl pH 4 | 4 | 70 | 100 |
| 8 | HCl pH 4 | 25 | 543 | 98 |
| 9 | HCl pH 4 | 37 | 401 | 98 |
| 10 | NaOH pH 10 | 4 | 22941 | 0 |
| 11 | NaOH pH 10 | 25 | 19875 | 1 |
| 12 | NaOH pH 10 | 37 | 20681 | 0 |

| **CONTROL NO SURFACE ADDED** | | | | |
|---|---|---|---|---|
| 13 | | | 20802 | |

### Conclusions

Water and HCl used in this experiment are both acidic and gave better binding of nucleic acid to hydrated zirconium silicate composition as compared to the other binding buffers, both of which are basic. Due to the similar nucleic acid purification properties of the other hydrated compositions as shown in Example 4, it is expected that acidic conditions would favorable effect binding of nucleic acid to such other hydrated compositions

Temperature, at least under conditions of this experiment, did not seem to affect binding.

### EXAMPLE 8

### Decreasing pH Effect on Elution of Nucleic Acid from Hydrated Compositions

This experiment was performed to determine if lowering the pH of the elution buffer will effect the efficiency of DNA elution for hydrated zirconium silicate compositions. Previously, it was shown that raising the pH had a positive effect on DNA elution from these surfaces (Example 5).

Two acids were tested at two different pH values each. Specifically, 5mg of each surface was added to 200uL of water followed by 1uL of ³²P labeled DNA (10⁴ genomic copies of *M. tuberculosis* DNA). The resulting suspension was incubated on a rotator device for 30 minutes. Following centrifugation, the supernate was removed and added to 10mL scintillation fluid for counting. 200uL of the elution buffer was added to the remaining pellet and the resulting suspension was incubated at 65°C for 30 minutes. Following centrifugation the supernate was removed and counted as above. The results are shown below.

| **REACTION NO.** | **SURFACE** | **ELUTION BUFFER** | **CPM SUPER BINDING** | **% DNA BOUND** | **CPM SUPER ELUTION** |
|---|---|---|---|---|---|
| 1 | Hyd ZrSiO₄ 080596b | H20 | 1581 | 90 | 730 |
| 2 | | HCl pH 4 | 134 | 100 | 1173 |
| 3 | | HCl pH 2 | 371 | 98 | 184 |
| 4 | | H3PO4 pH 4 | 319 | 98 | 830 |
| 5 | | H3PO4 pH 2 | 439 | 97 | 1002 |
| 6 | Hyd ZrSiO₄ (7-17-96) (SFSF)b | H20 | 515 | 96 | 1462 |
| 7 | | HCl pH 4 | 626 | 95 | 697 |
| 8 | | HCl pH 2 | 351 | 98 | 936 |
| 9 | | H3PO4 pH 4 | 295 | 98 | 1353 |
| 10 | | H3PO4 pH 2 | 515 | 96 | 788 |
| | Hyd ZrSiO₄ | | | | |
| 11 | 7-8-96545a | H20 | 2489 | 86 | 2154 |
| 12 | | HCl pH 4 | 2322 | 85 | 1189 |
| 13 | | HCl pH 2 | 2547 | 87 | 1139 |
| 14 | | H3PO4 pH 4 | 2961 | 83 | 1784 |
| 15 | | H3PO4 pH 2 | 2912 | 83 | 1183 |

| **CONTROL NO SURFACE ADDED** | | | | | |
|---|---|---|---|---|---|
| 16 | | | 16906 | | |

### Conclusions

Lowering the pH appears not to affect the amount of *M. tuberculosis* eluted compared to water. It may, however, be useful for getting DNA to bind to the composition. Also, binding of DNA to the compositions is very reproducible. Similar results would be expected with other hydrated compositions based on the similar nucleic acid purification properties shown in Example 4.

While the invention has been described with some specificity, modifications apparent to those with ordinary skill in the art may be made without departing from the scope of the invention. Various features of the invention are set forth in the following claims.

## Claims

1. A composition which binds nucleic acid, said composition selected from the group consisting of hydrated zirconium oxide, hydrated hafnium oxide, hydrated aluminum oxide, zirconium oxide, hafnium oxide and aluminum oxide.

2. The composition of claim 1 which binds nucleic acid in water.

3. The composition of claim 1 having a density of at least 1.0mg/uL.

4. The composition of claim 1 which in an amount of 2.5 milligrams will bind at least about 85 percent of 104 copies of a target nucleic acid in a sample without other nucleic acids.

5. A process for purifying nucleic acid from a sample comprising the steps of:
(a) exposing the sample to the composition of claim 1 for a period of time sufficient for nucleic acid in the sample to bind to said composition; and
(b) separating said composition from the sample.

6. The process of claim 6 wherein the separation of composition from sample is conducted by centrifugation or filtration.

7. The process of claim 6 further comprising:
(c) recovering nucleic acid from said composition.

8. A kit for purifying nucleic acid from a sample comprising the composition of claim 1.

9. The kit of claim 8 wherein said composition is present as particles.

10. A process for binding and subsequently eluting nucleic acid from a surface capable of binding said nucleic acid comprising the steps of:
(a) contacting said nucleic acid with said surface in the presence of a buffer solution with a pH below about 7.0; and
(b) eluting said nucleic acid from said surface in the presence of a buffer solution with a pH above about 7.0.
